# EUROPEAN PATENT APPLICATION

(11) **EP 0 791 362 A2**
(43) Date of publication of application: **27.08.1997**
(21) Application number: 96870147.4
(22) Date of filing: 13.11.1996
(51) Int. Cl.: A61L 2/16, C11D 3/00

(54) **Disinfecting compositions and processes for disinfecting surfaces**

(30) Priority: 23.02.1996 EP 96870018
(71) Applicant: THE PROCTER & GAMBLE COMPANY, Cincinnati, Ohio 45202 (US)
(72) Inventor: Romano, Nicoletta (NMN), 00144 Rome (IT); Trani, Marina (NMN), 00136 Roma (IT)
(74) Representative: Engisch, Gautier

(57) **Abstract**

The present invention relates to a disinfecting composition comprising a peroxygen bleach, from 0.003% to 5% by weight of the total composition of a cyclic terpene or a derivative thereof and from 0.003% to 5% by weight of the total composition of a phenolic compound, as defined herein. In another embodiment the present invention relates to a disinfecting composition comprising from 0.003% to 5% by weight of the total composition of a cyclic terpene or a derivative thereof and from 0.003% to 5% by weight of the total composition of eugenol, carvacrol and/or ferulic acid, said composition being free of a peroxygen bleach. The present invention also encompasses a process of disinfecting an inanimate surface wherein a composition with or without a peroxygen bleach, and comprising from 0.003% to 5% by weight of a cyclic terpene or a derivative thereof, and from 0.003% to 5% by weight of a phenolic compound, as defined herein, is applied onto said surface.

## Description

### Technical field

The present invention relates to compositions which can be used to disinfect various surfaces including animate surfaces (e.g., human skin, mouth, and the like) and inanimate surfaces including, but not limited to, hard surfaces like walls, tiles, floors, glass, bathroom surfaces, kitchen surfaces, dishes as well as fabrics, clothes, carpets and the like.

### Background

Antimicrobial compositions contain materials which have the ability to disinfect. It is generally recognised that a disinfecting material greatly reduces or even eliminates the microorganisms existing on a surface. Compositions based on bleaches like hypochlorite bleach, or on quaternary ammonium compounds have been extensively described in the art for disinfecting purpose.

Although such disinfecting compositions provide good disinfecting properties they do not encounter good acceptance amongst the consumers who are looking for disinfecting compositions based on safer and natural compounds.

It is thus an object of the present to provide effective disinfecting performance onto various surfaces while being safe to the surfaces treated and the environment.

It has now been found that this can be achieved by combining two particular classes of antimicrobial actives, namely a cyclic terpene and/or a derivative thereof with a phenolic compound, as defined herein after. Indeed, it has surprisingly been found that the use of a cyclic terpene and/or a derivative thereof (e.g., eucalyptol) together with a phenolic compound, as defined herein, (e.g., eugenol, carvacrol, ferulic acid) results in a synergistic effect on disinfecting performance. Advantageously, effective disinfecting performance is provided using a low total level of antimicrobial compounds.

Accordingly, in one embodiment the present invention encompasses a peroxygen bleach free disinfecting composition comprising from 0.003% to 5% by weight of the total composition of a cyclic terpene or a derivative thereof and from 0.003% to 5% by weight of eugenol, carvacrol and/or ferulic acid. In another embodiment the present invention encompasses a peroxygen bleach-containing disinfecting composition comprising from 0.003% to 5% by weight of a cyclic terpene or a derivative thereof and from 0.003% to 5% by weight of a phenolic compound, as defined herein.

An advantage of the present compositions is that they may be used to provide effective disinfection on any surfaces, even at high dilution levels, i.e., up to dilution levels of from 1:100 (composition:water).

Another advantage of the present invention is that effective disinfection is provided on a broad range of pure bacterial strains including Gram positive and Gram negative bacterial strains and more resistant micro-organisms like fungi. The combination of antimicrobial actives according to the present invention is particularly effective on Gram negative bacterial strains.

A further advantage of the compositions according to the present invention, when formulated in liquid aqueous form, is that they exhibit excellent chemical stability upon prolonged storage periods. This is particularly noticeable in the embodiment herein wherein the liquid aqueous disinfecting compositions of the present invention comprise a peroxygen bleach. For example a liquid aqueous peroxygen bleach-containing composition comprising the two particular classes of antimicrobial actives as defined herein, exhibits improved stability as compared to the same composition comprising antimicrobial essential oils instead of said antimicrobial actives.

Yet another advantage of the compositions according to the present invention is that, besides the disinfecting properties delivered, good cleaning is also provided, especially in the embodiment of the present invention where the compositions herein further comprise surfactants and/or solvents.

The compositions of the present invention are suitable for disinfecting various surfaces including animate surfaces (e.g. skin, mouth as for example liquid mouthwash compositions or toothpastes) and more particularly inanimate surfaces. Indeed, this technology finds a preferred application in hard-surfaces applications, laundry applications, e.g., in a so-called "soaking mode", "through the wash mode" and/or "pretreatment mode", as well as in carpet applications.

Accordingly, in another embodiment the present invention encompasses a process of disinfecting an inanimate surface wherein a composition with or without a peroxygen bleach and comprising from 0.003% to 5% by weight of the total composition of a cyclic terpene or a derivative thereof, and from 0.003% to 5% by weight of a phenolic compound, as defined herein, is applied onto said surface.

### Background art

Representative of the prior art is for example WO88/00795 which discloses liquid disinfecting compositions comprising a compound selected from the group of organic acids, perborates, peracids and their salts, together with other antimicrobial compounds like quaternary ammonium salts and essential oils.

The two particular classes of antimicrobial actives, as defined herein, are not disclosed.

US-5,403,587 discloses aqueous antimicrobial compositions which can be used to sanitise, disinfect, and clean hard-surfaces. More particularly, US-5,403,587 discloses aqueous compositions (pH 1 to 12) comprising essential oils (0.02% to 5%), which exhibit antimicrobial properties efficacy such as thyme oil, eucalyptus oil, clove oil and the like, and a solubilizing or dispersing agent sufficient to form an aqueous solution or dispersion of said essential oils in a water carrier. The two particular classes of antimicrobial actives, as defined herein, are not disclosed let alone the synergistic effect on disinfecting performance associated thereto.

WO 96/16633 discloses antimicrobial mouthwash compositions comprising a polyol, surfactant, alcohol, thymol and a compound selected from the group of eucalyptol, menthol, methyl salicylate. No peroxygen bleaches are disclosed. No eugenol is disclosed.

### Summary of the invention

The present invention encompasses a disinfecting composition comprising from 0.003% to 5% by weight of the total composition of a cyclic terpene or a derivative thereof and from 0.003% to 5% by weight of eugenol, carvacrol and/or ferulic acid, said composition being free of a peroxygen bleach.

In another embodiment the present invention encompasses a peroxygen bleach-containing disinfecting composition comprising from 0.003% to 5% by weight of the total composition of a cyclic terpene or a derivative thereof and from 0.003% to 5% by weight of a phenolic compound according to the formula: 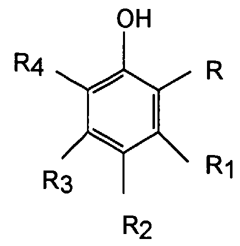 wherein R, R1, R2, R3, R4 independently are H, a linear or branched, saturated or unsaturated hydrocarbon chain of from 1 to 20 carbon atoms, an alkoxylated hydrocarbon chain according to the formula Ra(A)ₙ wherein Ra is a linear or branched, saturated or unsaturated hydrocarbon chain of from 1 to 20 carbon atoms, wherein A is butoxy, propoxy and/or ethoxy, and n is an integer of 1 to 4, or an aryl chain from 1 to 20 carbon atoms.

Furthermore, the present invention also encompasses such disinfecting compositions in their liquid form, packaged in a spray dispenser as well as a wipe incorporating such disinfecting compositions.

In yet another embodiment the present invention encompasses a process of disinfecting an inanimate surface with a composition being selected from the group consisting of:
- a composition comprising from 0.003% to 5% by weight of the total composition of a cyclic terpene or a derivative thereof and from 0.003% to 5% by weight of a phenolic compound according to the formula: 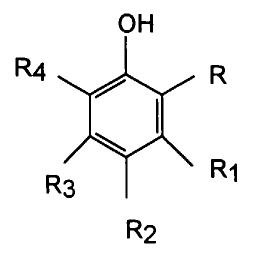 wherein R, R1, R2, R3, R4 independently are H, a linear or branched, saturated or unsaturated hydrocarbon chain of from 1 to 20 carbon atoms, an alkoxylated hydrocarbon chain according to the formula Ra(A)ₙ wherein Ra is a linear or branched, saturated or unsaturated hydrocarbon chain of from 1 to 20 carbon atoms, wherein A is butoxy, propoxy and/or ethoxy, and n is an integer of 1 to 4, or an aryl chain from 1 to 20 carbon atoms, said composition being free of a peroxygen bleach, and
- a composition comprising a peroxygen bleach, from 0.003% to 5% by weight of the total composition of a cyclic terpene or a derivative thereof and from 0.003% to 5% by weight of a phenolic compound according to the formula: 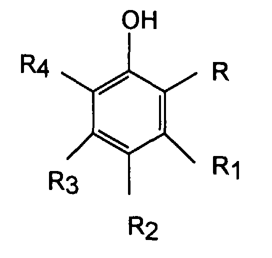 wherein R, R1, R2, R3, R4 independently are H, a linear or branched, saturated or unsaturated hydrocarbon chain of from 1 to 20 carbon atoms, an alkoxylated hydrocarbon chain according to the formula Ra(A)ₙ wherein Ra is a linear or branched, saturated or unsaturated hydrocarbon chain of from 1 to 20 carbon atoms, wherein A is butoxy, propoxy and/or ethoxy, and n is an integer of 1 to 4, or an aryl chain from 1 to 20 carbon atoms,
said process comprising the step of applying said composition onto said surface.

### Detailed description of the invention

### The compositions:

The present invention is based on the finding that the combination of a cyclic terpene and/or a derivative thereof together with a phenolic compound, as defined herein, results in a synergistic effect on disinfecting performance. Thus, an advantage of the present invention is to get effective disinfecting performance with low total level of antimicrobial actives.

A further advantage of said antimicrobial actives is that they may impart pleasant odor to the disinfecting compositions of the present invention without the need of adding a perfume. Indeed, the disinfecting compositions according to the present invention deliver not only effective disinfecting properties on surfaces to be disinfected but also good scent.

Effective disinfecting performance is obtained with the compositions according to the present inventions on a variety of microorganisms including Gram negative bacteria like *Pseudomonas aeroginosa*, *Escherichia coli* as well as on fungi like *Candida albicans* present on infected surfaces, even if used in highly diluted conditions, e.g. up to a dilution level of 1:100 (composition:water).

The disinfecting properties of a composition according to the present invention may be measured by the bactericidal activity of said composition. A test method suitable to evaluate the bactericidal activity of a composition on infected surfaces is described in European Standard, prEN 1040, CEN/TC 216 N 78, dated November 1995 issued by the European committee for standardisation, Brussels. European Standard, prEN 1040, CEN/TC 216 N 78, specifies a test method and requirements for the minimum bactericidal activity of a disinfecting composition. The test is passed if the bacterical colonies forming units (cfu) are reduced from a 10⁷ cfu (initial level) to a 10² cfu (final level after contact with the disinfecting product), i.e. a 10⁵ reduction of the viability is necessary. The compositions according to the present invention pass this test, even if used in highly diluted conditions.

Accordingly, in one embodiment the present invention encompasses a disinfecting composition comprising a peroxygen bleach, from 0.003% to 5% by weight of the total composition of a cyclic terpene or a derivative thereof and from 0.003% to 5% by weight of a phenolic compound, as defined herein.

In another embodiment the present invention also encompasses a disinfecting composition comprising from 0.003% to 5% by weight of the total composition of a cyclic terpene or a derivative thereof and from 0.003% to 5% by weight of eugenol, carvacrol and/or ferulic acid, said composition being free of peroxygen bleach.

An essential ingredient of the compositions according to the present invention is a cyclic terpene, a derivative thereof or a mixture thereof. Cyclic terpenes, derivatives thereof or mixtures thereof are present at a level of 0.003% to 5% by weight of the total composition, preferably from 0.005% to 3%, and more preferably from 0.05% to 1%.

Suitable cyclic terpenes to be used herein include any mono- or polycyclic compound including at least one isoprenic unit, preferably 1 or 2, said isoprenic unit may comprise one or more hetero atoms, preferably oxygen, and said isoprenic unit may be substituted at any carbon by H, a linear or branched, saturated or unsaturated hydrocarbon chain having from 1 to 20 carbon atoms, preferably from 1 to 10, more preferably from 1 to 4, an alkoxylated hydrocarbon chain of the formula Ra(A)ₙ wherein Ra is a linear or branched, saturated or unsaturated hydrocarbon chain having from 1 to 20 carbon atoms, preferably from 1 to 10, more preferably from 1 to 4, wherein A is butoxy, propoxy and/or ethoxy, and n is an integer of from 1 to 4, preferably from 1 to 3, or an aryl chain having from 1 to 20 carbon atoms, preferably from 1 to 10 and more preferably from 1 to 4.

Particularly suitable cyclic terpenes include mono-and bicyclic monoterpenes, especially those of the hydrocarbon class, which include the terpinenes, terpinolenes, limonenes and pinenes and mixtures thereof. Preferred material of this type are d-limonene, dipentene, alpha-pinene and/or beta-pinene. For example pinene is commercially available from SXCM Glidco (jacksonville) under the name Alpha Pinene P&F®.

Suitable cyclic terpene derivatives to be used herein include alcohols, aldehydes, esters and ketones. Particularly suitable cyclic terpene derivatives are menthol, terpineol, borneol, eucalyptol, cedrol, camphor, carvone, cariophyllene, verbenone, ponocarvone and mixtures thereof.

Highly preferred cyclic terpenes or derivatives thereof to be used herein are limonene, menthol, eucalyptol, terpineol and mixtures thereof.

Another essential ingredient of the compositions according to the present invention is a phenolic compound or a mixture thereof. Phenolic compounds or mixtures thereof are present at a level of 0.003% to 5% by weight of the total composition, preferably from 0.005% to 3%, and more preferably from 0.05% to 1%.

The phenolic compounds are according to the formula: 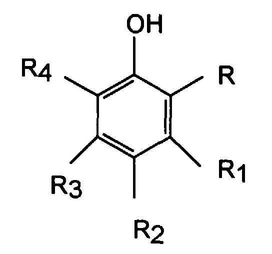 wherein R, R1, R2, R3, R4 are independently H, a linear or branched, saturated or unsaturated hydrocarbon chain having from 1 to 20 carbon atoms, preferably from 1 to 10, more preferably from 1 to 4, an alkoxylated hydrocarbon chain according to the formula Ra(A)ₙ wherein Ra is a linear or branched, saturated or unsaturated hydrocarbon chain having from 1 to 20 carbon atoms, preferably from 1 to 10, more preferably from 1 to 4, wherein A is butoxy, propoxy and/or ethoxy, and n is an integer of 1 to 4, preferably from 1 to 3, or an aryl chain having from 1 to 20 carbon atoms, preferably from 1 to 10 and more preferably from 1 to 4.

Suitable phenolic compounds to be used herein include eugenol, thymol, methyl salicylate, carvacrol, ferulic acid or mixtures thereof. Particularly preferred phenolic compounds are eugenol, carvacrol and/or ferulic acid and more preferred is eugenol.

Eugenol may be commercially available for example from Sigma, Systems - Bioindustries (SBI) - Manheimer Inc.

In the embodiment where the compositions of the present invention comprise a peroxygen bleach, or a mixture thereof, said peroxygen bleach is preferably hydrogen peroxide, or a water soluble source thereof, or a mixture thereof. Hydrogen peroxide is most preferred to be used in the compositions according to the present invention.

It is believed that the presence of said peroxygen bleach especially hydrogen peroxide in the compositions according to the present invention further contribute to the disinfecting properties of said compositions. Indeed, said peroxygen bleach may attack the vital function of the microorganism cells, for example, it may inhibit the assembling of ribosomes units within the cytoplasm of the microorganism cells. Also, said peroxygen bleach like hydrogen peroxide, is a strong oxidizer that generates hydroxyl free radicals which attack proteins and nucleic acids. Furthermore, the presence of said peroxygen bleach, especially hydrogen peroxide, provides strong stain removal benefits which are particularly noticeable for example in laundry and/or hard surfaces applications.

As used herein a hydrogen peroxide source refers to any compound which produces perhydroxyl ions when said compound is in contact with water. Suitable water-soluble sources of hydrogen peroxide for use herein include percarbonates, persilicates, persulphates such as monopersulphate, perborates, peroxyacids such as diperoxydodecandioic acid (DPDA), or magnesium perphthalic acid, dialkylperoxides, diacylperoxides, preformed percarboxylic acids, organic/ inorganic peroxides and/or hydroperoxides or mixtures thereof.

In the embodiment of the present invention where peroxygen bleach or a mixture thereof is present in the compositions of the present invention, they comprise at least 0.01 % by weight of the total composition of said peroxygen bleach, or mixtures thereof, preferably from 0.1% to 15%, and more preferably from 1% to 10%.

The compositions according to the present invention may be formulated either as solids (e.g., powder/granular) or liquids. Preferred compositions according to the present invention are liquid compositions, including aqueous compositions and non-aqueous compositions. As used herein, "liquid" includes "pasty" compositions, and liquid compositions herein preferably have a viscosity of from 1 cps to 10000 cps, preferably from 100 cps to 1000 cps, more preferably from 200 cps to 600 cps when measured with a Carrimed Rheometer at a temperature of 25°C and a shear rate of 15-35 s⁻¹. Aqueous compositions are preferred herein.

Typically, the liquid compositions of the present invention have a pH as is of not more than 12.0, more preferably from 2 to 10, and most preferably from 3 to 9. The pH of the compositions can be adjusted by using organic or inorganic acids, or alkalinising agents.

Also an advantage of the compositions of the present invention which are formulated in a liquid aqueous form is that they are chemically stable upon prolonged periods of storage. More particularly, the liquid aqueous compositions of the present invention comprising a peroxygen bleach, the cyclic terpene and/or a derivative thereof and the phenolic compound, as defined herein, exhibit improved chemical stability upon prolonged storage periods while delivering effective disinfecting performance on surfaces, even when used upon highly diluted conditions, as compared to the chemical stability obtained with the same compositions comprising the corresponding antimicrobial essential oils instead of said antimicrobial actives.

Chemical stability of the compositions herein may be evaluated by measuring the concentration of available oxygen (often abbreviated to Avox) at given storage time after having manufactured the compositions. The concentration of available oxygen can be measured by chemical titration methods known in the art, such as the iodimetric method, thiosulphatimetric method, the permanganometric method and the cerimetric method. Said methods and the criteria for the choice of the appropriate method are described for example in "Hydrogen Peroxide", W. C. Schumb, C. N. Satterfield and R. L. Wentworth, Reinhold Publishing Corporation, New York, 1955 and "Organic Peroxides", Daniel Swern, Editor Wiley Int. Science, 1970.

### Processes of disinfecting inanimate surfaces:

The present invention encompasses a process of disinfecting an inanimate surface with a composition being selected from the group consisting of:
- a composition comprising from 0.003% to 5% by weight of the total composition of a cyclic terpene or a derivative thereof, and from 0.003% to 5% by weight of the total composition of a phenolic compound, as defined herein, said composition being free of a peroxygen bleach, and
- a composition comprising a peroxygen bleach, from 0.003% to 5% by weight of the total composition of a cyclic terpene or a derivative thereof, and from 0.003% to 5% by weight of the total composition of a phenolic compound, as defined herein,
said process comprising the step of applying said composition onto said surface.

Cyclic terpenes and/or derivatives thereof, phenolic compounds, peroxygen bleaches are as defined herein before.

It has now surprisingly been found that there is a synergistic effect on disinfection associated to the combination of a cyclic terpene and/or a derivative thereof and a phenolic compound, as defined herein, when used to disinfect inanimate surfaces.

By "inanimate surfaces" it is meant herein any inert hard or soft surface.

These inanimate surfaces include, but are not limited to, hard-surfaces typically found in houses like kitchens, bathrooms, or in car interiors, e.g., tiles, walls, floors, chrome, glass, smooth vinyl, any plastic, plastified wood, table top, sinks, cooker tops, dishes, sanitary fittings such as sinks, showers, shower curtains, wash basins, WCs and the like, as well as fabrics including clothes, curtains, drapes, bed linens, bath linens, table cloths, sleeping bags, tents, upholstered furniture and the like, and carpets. Inanimate surfaces also include household appliances including, but not limited to, refrigerators, freezers, washing machines, automatic dryers, ovens, microwave ovens, dishwashers and so on.

In one embodiment of the present invention the process of disinfecting herein is directed particularly to fabrics. In such a process a disinfecting composition, as described herein, needs to be contacted with the fabrics to be disinfected. This can be done either in a so-called "pretreatment mode", where the composition being in its liquid form is applied neat onto said fabrics before the fabrics are rinsed, or washed then rinsed, or in a "soaking mode" where the composition being either in its solid form or liquid form, is first diluted in an aqueous bath and the fabrics are immersed and soaked in the bath, before they are rinsed, or in a "through the wash mode", where the composition being either in its solid form or liquid form, is added on top of a wash liquor formed by dissolution or dispersion of a typical laundry detergent.

In the pretreatment mode, it has been found that it is highly preferred that the fabrics be rinsed after they have been contacted with a disinfecting composition, as described herein, in its liquid form, before said composition has completely dried off, especially in the embodiment herein wherein the disinfecting composition used comprises peroxygen bleach on top of the cyclic terpene and phenolic compound as defined herein. Indeed, it has been found that water evaporation contributes to increase the concentration of free radicals onto the surface of the fabrics and, consequently, the rate of chain reaction. Indeed, free radicals typically result from the decomposition of peroxygen bleach that may be catalysed due to the presence of metal ions on the surface of a fabric and/or to the exposure of the fabrics to UV radiation from sunlight. It is also speculated that an auto-oxidation reaction occurs upon evaporation of water when liquid peroxygen-bleach containing compositions are left to dry onto the fabrics. Said reaction of auto-oxidation generates peroxy-radicals which may contribute to the degradation of cellulose. Thus, not leaving the liquid peroxygen bleach-containing compositions, as described herein, to dry onto the fabric, in a process of pretreating fabrics, contributes to reduce the tensile strength loss when pretreating fabrics with liquid peroxygen bleach-containing compositions.

In the pretreatment mode, the process comprises the steps of applying a disinfecting composition, as described herein, in its liquid form neat onto said fabrics, or at least infected portions thereof (i.e., directly applying said liquid composition as described herein onto said fabrics without undergoing any dilution), and subsequently rinsing, or washing then rinsing said fabrics. In this mode, the neat compositions can optionally be left to act onto said fabrics for a period of time ranging from 1 min. to 1 hour, preferably from 1 minute to 30 minutes, before the fabrics are rinsed, or washed then rinsed, provided that the composition is not left to dry onto said fabrics. For particularly though stains, it may be appropriate to further rub or brush said fabrics by means of a sponge or a brush, or by rubbing two pieces of fabrics against each other.

In another mode, generally referred to as "soaking", the process comprises the steps of diluting a disinfecting composition as described herein being in its liquid or solid form in an aqueous bath so as to form a diluted composition. The dilution level of said disinfecting composition in an aqueous bath is typically up to 1:85, preferably up to 1:50 and more preferably about 1:25 (composition:water). The fabrics are then contacted with the aqueous bath comprising the disinfecting composition, and the fabrics are finally rinsed, or washed then rinsed. Preferably in that embodiment, the fabrics are immersed in the aqueous bath comprising the composition, and also preferably, the fabrics are left to soak therein for a period of time ranging from 1 minute to 48 hours, preferably from 1 minute to 24 hours.

In yet another mode which can be considered as a sub-embodiment of "soaking", generally referred to as "through the wash mode", the disinfecting composition as described herein, in its liquid or solid form is used as a so-called laundry additive. And in that embodiment the aqueous bath is formed by dissolving or dispersing a conventional laundry detergent in water. The disinfecting composition is contacted with the aqueous bath, and the fabrics are then contacted with the aqueous bath containing the disinfecting composition. Finally, the fabrics are rinsed.

In another embodiment the process of disinfecting herein is directed particularly to carpets. In such a process a disinfecting composition, as described herein, needs to be contacted with the carpets to be disinfected. Thus, the present invention also encompasses a process of disinfecting carpets with a disinfecting composition as described herein, wherein said process comprises the step of applying said composition to said carpet, then optionally rubbing and/or brushing said composition into said carpet, preferably only infected portions thereof before removing said composition from said carpet.

An advantage of the present invention is that the disinfecting action of the disinfecting composition as described herein commence as soon as the composition is applied onto said carpet. Thus, the disinfecting process of the present invention does not necessarily require rubbing and/or brushing. It is only in the case of highly infected carpets that the carpet may be disinfected by applying onto it the disinfecting composition according to the present invention, then rubbing and/or brushing it more or less intensively for example by means of a sponge or a brush or other mechanical/electrical device, optionally with the aid of water. In general the rubbing/brushing-times are between 1 to a few minutes per square meter. After the disinfecting composition according to the present invention has been applied onto the carpet and optionally rubbed and/or brushed, the composition is removed from said carpet, preferably by mechanical means including brushing out and/or vacuum cleaning.

As for the "soaking mode" and "through the wash mode" when disinfecting a fabric, the compositions for the disinfection of carpets according to the present invention may be applied to the carpet to be disinfected either in neat or diluted form, this applies to compositions being either liquid compositions or solid compositions (e.g. powder or granular compositions).

By "diluted form" it is meant herein that the carpet disinfecting compositions according to the present invention may be diluted by the user, preferably with water. Said compositions can be diluted up to 100 times, preferably up to 50 times and more preferably up to 25 times.

In a preferred embodiment herein, the compositions for the disinfection of carpets according to the present invention are liquid aqueous compositions. Indeed, a liquid aqueous composition, i.e. an aqueous disinfecting composition as described herein, in its neat form or which has been diluted with water by the user, or an aqueous composition resulting from the dilution of a granular composition or of a powder composition, is applied to the carpet to be disinfected, said carpet is optionally rubbed and/or brushed, then said composition is left to dry and then removed from said carpet. Indeed, said liquid aqueous composition is left to dry until said composition which combined with dirt has been changed into dry residues. These residues are then removed from the carpet mechanically. Such liquid aqueous compositions may be applied directly onto the area to be treated or applied using a cloth or piece of material such as spraying device or aerosol can, a sponge, a brush or other mechanical/electrical device. In a preferred embodiment of the invention the liquid aqueous disinfecting composition as described herein is applied to the area to be treated by using a spraying device or an aerosol can. Such a spraying device may be trigger operated or pump operated or electrically operated or operated by any source of pressurised gas such as a can or a pressurizer. Such spraying devices are particularly preferable if a large area is to be treated as it facilitates the ease of use for the consumer. The spraying devices ensure uniform coverage of the area to be treated and maximises the advantage of using the liquid aqueous compositions according to the present invention. This is because the application of product by spray best allows the product to be left to dry on the area treated, even without rubbing or brushing. This optimises the action time of the composition and allows the best exploitation of the disinfecting action of the antimicrobial actives therein, and peroxygen bleach if present.

In another embodiment, the compositions for the disinfection of carpets according to the present invention are solid compositions (e.g., granular or powder compositions). Such solid disinfecting compositions according to the present invention may be applied directly onto the area of the carpet to be treated by for example sprinkling said composition over said area or may be applied by using a sponge, a brush, or other mechanical/electrical device preferably in presence of water and then left to dry and then removed from said carpet.

The area to be treated using the disinfecting compositions according to the present invention may be any size. In addition a complete section or even a whole carpet may be applied with the composition for the disinfection of carpets according to the present invention. For such purposes when using a liquid aqueous composition a spraying device with a pump to allow prolonged spraying is particularly useful.

The amount of the compositions for the disinfection of carpets according to the present invention applied will depend on the severity of the infected stains/soils. And advantage of the disinfecting process herein is that it also to deodorise the carpets.

The disinfecting compositions as described herein for the disinfection of carpets according to the present invention may be used both for manual carpet operation and for carpet machines. For carpet machines the disinfecting compositions according to the present invention, i.e. either liquid compositions or solid compositions, may be preferably diluted according to the machine operating instructions.

In yet another embodiment the process of disinfecting herein is directed particularly to hard-surfaces. In such a process a disinfecting composition, as described herein, needs to be contacted with the hard-surfaces to be disinfected. Thus, the present invention also encompasses a process of disinfecting a hard-surface with a disinfecting composition as described herein, wherein said process comprises the step of applying said composition to said hard-surface, preferably only infected portions thereof and optionally rinsing said hard-surface.

In the process of disinfecting hard-surfaces according to the present invention the disinfecting compositions as described herein are in liquid form and may be applied to the surface to be disinfected in their neat form or in their diluted form typically at a dilution level up to 100 times their weight of water, preferably into 80 to 2 times their weight of water, and more preferably 60 to 10 times.

In the preferred embodiment of the process of the present invention wherein said liquid composition is applied to a hard-surface to be disinfected in its diluted form, it is not necessary to rinse the surface after the composition has been applied, indeed no visible residues are left onto the surface.

### Optional ingredients

The compositions according to the present invention may further comprise a surfactant or mixtures thereof. Suitable surfactants to be used herein may be any surfactant known to those skilled in the art including anionic, nonionic, cationic, amphoteric and/or zwitterionic surfactants. Surfactants contribute to the cleaning performance of a composition comprising a cyclic terpene and a phenolic compound, as defined herein.

Particularly suitable anionic surfactants to be used herein include water soluble salts or acids of the formula ROSO₃M wherein R is preferably a C₆-C₂₄ hydrocarbyl, preferably an alkyl or hydroxyalkyl having a C₁₀-C₂₀ alkyl component, more preferably a C₁₂-C₁₈ alkyl or hydroxyalkyl, and M is H or a cation, e.g., an alkali metal cation (e.g., sodium, potassium, lithium), or ammonium or substituted ammonium (e.g., methyl-, dimethyl-, and trimethyl ammonium cations and quaternary ammonium cations, such as tetramethylammonium and dimethyl piperdinium cations and quaternary ammonium cations derived from alkylamines such as ethylamine, diethylamine, triethylamine, and mixtures thereof, and the like).

Other suitable anionic surfactants to be used herein include alkyl-diphenylether-sulphonates and alkyl-carboxylates. Other anionic surfactants can include salts (including, for example, sodium, potassium, ammonium, and substituted ammonium salts such as mono-, di- and triethanolamine salts) of soap, C₉-C₂₀ linear alkylbenzenesulfonates, C₈-C₂₂ primary or secondary alkanesulfonates, C₈-C₂₄ olefinsulfonates, sulfonated polycarboxylic acids prepared by sulfonation of the pyrolyzed product of alkaline earth metal citrates, e.g., as described in British patent specification No. 1,082,179, C₈-C₂₄ alkylpolyglycolethersulfates (containing up to 10 moles of ethylene oxide); alkyl ester sulfonates such as C₁₄₋₁₆ methyl ester sulfonates; acyl glycerol sulfonates, fatty oleyl glycerol sulfates, alkyl phenol ethylene oxide ether sulfates, paraffin sulfonates, alkyl phosphates, isethionates such as the acyl isethionates, N-acyl taurates, alkyl succinamates and sulfosuccinates, monoesters of sulfosuccinate (especially saturated and unsaturated C₁₂-C₁₈ monoesters) diesters of sulfosuccinate (especially saturated and unsaturated C₆-C₁₄ diesters), acyl sarcosinates, sulfates of alkylpolysaccharides such as the sulfates of alkylpolyglucoside (the nonionic nonsulfated compounds being described below), branched primary alkyl sulfates, alkyl polyethoxy carboxylates such as those of the formula RO(CH₂CH₂O)ₖCH₂COO-M⁺ wherein R is a C₈-C₂₂ alkyl, k is an integer from 0 to 10, and M is a soluble salt-forming cation. Resin acids and hydrogenated resin acids are also suitable, such as rosin, hydrogenated rosin, and resin acids and hydrogenated resin acids present in or derived from tall oil. Further examples are given in "Surface Active Agents and Detergents" (Vol. I and II by Schwartz, Perry and Berch). A variety of such surfactants are also generally disclosed in U.S. Patent 3,929,678, issued December 30, 1975 to Laughlin, et al. at Column 23, line 58 through Column 29, line 23 (herein incorporated by reference).

Preferred anionic surfactants for use in the compositions herein are the alkyl benzene sulfonates, alkyl sulfates, alkyl alkoxylated sulfates, paraffin sulfonates and mixtures thereof.

Suitable amphoteric surfactants to be used herein include amine oxides having the following formula R₁R₂R₃NO wherein each of R1, R2 and R3 is independently a saturated substituted or unsubstituted, linear or branched hyudrocarbon chains of from 1 to 30 carbon atoms. Preferred amine oxide surfactants to be used according to the present invention are amine oxides having the following formula R₁R₂R₃NO wherein R1 is an hydrocarbon chain comprising from 1 to 30 carbon atoms, preferably from 6 to 20, more preferably from 8 to 16, most preferably from 8 to 12, and wherein R2 and R3 are independently substituted or unsubstituted, linear or branched hydrocarbon chains comprising from 1 to 4 carbon atoms, preferably from 1 to 3 carbon atoms, and more preferably are methyl groups. R1 may be a saturated substituted or unsubstituted, linear or branched hydrocarbon chain.

Suitable amine oxides for use herein are for instance natural blend C8-C10 amine oxides as well as C12-C16 amine oxides commercially available from Hoechst.

Suitable zwitterionic surfactants to be used herein contain both cationic and anionic hydrophilic groups on the same molecule at a relatively wide range of pH's. The typical cationic group is a quaternary ammonium group, although other positively charged groups like phosphonium, imidazolium and sulfonium groups can be used. The typical anionic hydrophilic groups are carboxylates and sulfonates, although other groups like sulfates, phosphonates, and the like can be used. A generic formula for some zwitterionic surfactants to be used herein is

R₁-N⁺(R₂)(R₃)R₄X⁻

wherein R₁ is a hydrophobic group; R₂ and R₃ are each C₁-C₄ alkyl, hydroxy alkyl or other substituted alkyl group which can also be joined to form ring structures with the N; R₄ is a moiety joining the cationic nitrogen atom to the hydrophilic group and is typically an alkylene, hydroxy alkylene, or polyalkoxy group containing from 1 to 10 carbon atoms; and X is the hydrophilic group which is preferably a carboxylate or sulfonate group. Preferred hydrophobic groups R₁ are alkyl groups containing from 1 to 24, preferably less than 18, more preferably less than 16 carbon atoms. The hydrophobic group can contain unsaturation and/or substituents and/or linking groups such as aryl groups, amido groups, ester groups and the like. In general, the simple alkyl groups are preferred for cost and stability reasons.

Highly preferred zwitterionic surfactants include betaine and sulphobetaine surfactants, derivatives thereof or mixtures thereof. Said betaine or sulphobetaine surfactants are preferred herein as, they help disinfection by increasing the permeability of the bacterial cell wall, thus allowing other active ingredients to enter the cell.

Furthermore, due to the mild action profile of said betaine or sulphobetaine surfactants, they are particularly suitable for the cleaning of delicate surfaces, e.g., delicate laundry or surfaces in contact with food and/or babies. Betaine and sulphobetaine surfactants are also extremely mild to the skin and/or surfaces to be treated.

Suitable betaine and sulphobetaine surfactants to be used herein are the betaine/sulphobetaine and betaine-like detergents wherein the molecule contains both basic and acidic groups which form an inner salt giving the molecule both cationic and anionic hydrophilic groups over a broad range of pH values. Some common examples of these detergents are described in U.S. Pat. Nos. 2,082,275, 2,702,279 and 2,255,082, incorporated herein by reference. Preferred betaine and sulphobetaine surfactants herein are according to the formula 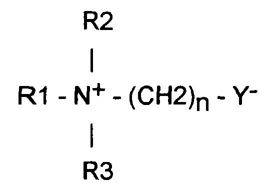 wherein R1 is a hydrocarbon chain containing from 1 to 24 carbon atoms, preferably from 8 to 18, more preferably from 12 to 14, wherein R2 and R3 are hydrocarbon chains containing from 1 to 3 carbon atoms, preferably 1 carbon atom, wherein n is an integer from 1 to 10, preferably from 1 to 6, more preferably is 1, Y is selected from the group consisting of carboxyl and sulfonyl radicals and wherein the sum of R1, R2 and R3 hydrocarbon chains is from 14 to 24 carbon atoms, or mixtures thereof.

Examples of particularly suitable betaine surfactants include C12-C18 alkyl dimethyl betaine such as coconut-betaine and C10-C16 alkyl dimethyl betaine such as laurylbetaine. Coconutbetaine is commercially available from Seppic under the trade name of Amonyl 265®. Laurylbetaine is commercially available from Albright & Wilson under the trade name Empigen BB/L®.

Other specific zwitterionic surfactants have the generic formulas:

R₁-C(O)-N(R₂)-(C(R₃)₂)ₙ-N(R₂)₂⁽⁺⁾-(C(R₃)₂)ₙ-SO₃⁽⁻⁾

or R₁-C(O)-N(R₂)-(C(R₃)₂)ₙ-N(R₂)₂⁽⁺⁾-(C(R₃)₂)ₙ-COO⁽⁻⁾

wherein each R₁ is a hydrocarbon, e.g. an alkyl group containing from 8 up to 20, preferably up to 18, more preferably up to 16 carbon atoms, each R₂ is either a hydrogen (when attached to the amido nitrogen), short chain alkyl or substituted alkyl containing from one to 4 carbon atoms, preferably groups selected from the group consisting of methyl, ethyl, propyl, hydroxy substituted ethyl or propyl and mixtures thereof, preferably methyl, each R₃ is selected from the group consisting of hydrogen and hydroxy groups and each n is a number from 1 to 4, preferably from 2 to 3, more preferably 3, with no more than one hydroxy group in any (C(R₃)₂) moiety. The R₁ groups can be branched and/or unsaturated. The R₂ groups can also be connected to form ring structures. A surfactant of this type is a C₁₀-C₁₄ fatty acylamidopropylene(hydroxypropylene)sulfobetaine that is available from the Sherex Company under the trade name "Varion CAS sulfobetaine"®.

In a preferred embodiment of the present invention where the compositions herein are particularly suitable for the disinfection of a hard-surface, the surfactant is typically a surfactant system comprising an amine oxide and a betaine or sulphobetaine surfactant, preferably in a weight ratio of amine oxide to betaine or sulphobetaine of 2:1 to 100:1, more preferably of 6:1 to 100:1 and most preferably 10:1 to 50:1. The use of such a surfactant system in the hard-surface compositions herein, provides effective cleaning performance and provides shine on the cleaned surfaces, i.e., the amount of filming/streaking left on the cleaned surface that has been treated with said compositions is minimal.

Suitable nonionic surfactants to be used herein are fatty alcohol ethoxylates and/or propoxylates which are commercially available with a variety of fatty alcohol chain lengths and a variety of ethoxylation degrees. Indeed, the HLB values of such alkoxylated nonionic surfactants depend essentially on the chain length of the fatty alcohol, the nature of the alkoxylation and the degree of alkoxylation. Surfactant catalogues are available which list a number of surfactants, including nonionics, together with their respective HLB values.

Particularly suitable for use herein as nonionic surfactants are hydrophobic nonionic surfactants having an HLB (hydrophilic-lipophilic balance) below 16, preferably below 15, more preferably below 12, and most preferably below 10. Those hydrophobic nonionic surfactants have been found to provide good grease cutting properties.

Preferred hydrophobic nonionic surfactants to be used in the compositions according to the present invention are surfactants having an HLB below 16 and being according to the formula RO-(C₂H₄O)ₙ(C₃H₆O)ₘH, wherein R is a C₆ to C₂₂ alkyl chain or a C₆ to C₂₈ alkyl benzene chain, and wherein n+m is from 0 to 20 and n is from 0 to 15 and m is from 0 to 20, preferably n+m is from 1 to 15 and, n and m are from 0.5 to 15, more preferably n+m is from 1 to 10 and, n and m are from 0 to 10. The preferred R chains for use herein are the C₈ to C₂₂ alkyl chains. Accordingly, suitable hydrophobic nonionic surfactants for use herein are Dobanol ^{R} 91-2.5 (HLB= 8.1; R is a mixture of C9 and C₁₁ alkyl chains, n is 2.5 and m is 0), or Lutensol ^{R} TO3 (HLB=8; R is a C₁₃ alkyl chains, n is 3 and m is 0), or Lutensol ^{R} AO3 (HLB=8; R is a mixture of C₁₃ and C₁₅ alkyl chains, n is 3 and m is 0), or Tergitol ^{R} 25L3 (HLB= 7.7; R is in the range of C₁₂ to C₁₅ alkyl chain length, n is 3 and m is 0), or Dobanol ^{R} 23-3 (HLB=8.1; R is a mixture of C₁₂ and C₁₃ alkyl chains, n is 3 and m is 0), or Dobanol ^{R} 23-2 (HLB=6.2; R is a mixture of C₁₂ and C₁₃ alkyl chains, n is 2 and m is 0), or Dobanol ^{R} 45-7 (HLB=11.6; R is a mixture of C₁₄ and C₁₅ alkyl chains, n is 7 and m is 0) Dobanol ^{R} 23-6.5 (HLB=11.9; R is a mixture of C₁₂ and C₁₃ alkyl chains, n is 6.5 and m is 0), or Dobanol ^{R} 25-7 (HLB=12; R is a mixture of C₁₂ and C₁₅ alkyl chains, n is 7 and m is 0), or Dobanol ^{R} 91-5 (HLB=11.6; R is a mixture of C₉ and C₁₁ alkyl chains, n is 5 and m is 0), or Dobanol ^{R} 91-6 (HLB=12.5 ; R is a mixture of C₉ and C₁₁ alkyl chains, n is 6 and m is 0), or Dobanol ^{R} 91-8 (HLB=13.7 ; R is a mixture of C₉ and C₁₁ alkyl chains, n is 8 and m is 0), Dobanol ^{R} 91-10 (HLB=14.2 ; R is a mixture of C₉ to C₁₁ alkyl chains, n is 10 and m is 0), or mixtures thereof. Preferred herein are Dobanol ^{R} 91-2.5 , or Lutensol ^{R} TO3, or Lutensol ^{R} AO3, or Tergitol ^{R} 25L3, or Dobanol ^{R} 23-3, or Dobanol ^{R} 23-2, or mixtures thereof. These Dobanol^{R} surfactants are commercially available from SHELL. These Lutensol^{R} surfactants are commercially available from BASF and these Tergitol ^{R} surfactants are commercially available from UNION CARBIDE.

Typically, the surfactant or mixtures thereof is present in the composition of the present invention at a level of from 0.01 % to 50% by weight of the total composition, preferably from 0.01% to 30% and more preferably from 0.1% to 20%.

The compositions according to the present invention may comprise as a preferred optional ingredient further antimicrobial ingredients that contribute to the antimicrobial activity of the compositions of the present invention. Such ingredients may be present up to a level of 5% by weight of the total composition, preferably from 0.001% to 1% and include parabens like ethyl paraben, propyl paraben, methyl paraben, glutaraldehyde or mixtures thereof.

The compositions herein may further comprise a chelating agent as a preferred optional ingredient. Suitable chelating agents may be any of those known to those skilled in the art such as the ones selected from the group comprising phosphonate chelating agents, amino carboxylate chelating agents or other carboxylate chelating agents, or polyfunctionally-substituted aromatic chelating agents or mixtures thereof.

Such phosphonate chelating agents may include etidronic acid (1-hydroxyethylidene-bisphosphonic acid or HEDP) as well as amino phosphonate compounds, including amino alkylene poly (alkylene phosphonate), alkali metal ethane 1-hydroxy diphosphonates, nitrilo trimethylene phosphonates, ethylene diamine tetra methylene phosphonates, and diethylene triamine penta methylene phosphonates. The phosphonate compounds may be present either in their acid form or as salts of different cations on some or all of their acid functionalities. Preferred phosphonate chelating agents to be used herein are diethylene triamine penta methylene phosphonates. Such phosphonate chelants are commercially available from Monsanto under the trade name DEQUEST^{®}.

Polyfunctionally-substituted aromatic chelating agents may also be useful in the compositions herein. See U.S. patent 3,812,044, issued May 21, 1974, to Connor et al. Preferred compounds of this type in acid form are dihydroxydisulfobenzenes such as 1,2-dihydroxy -3,5-disulfobenzene.

A preferred biodegradable chelating agent for use herein is ethylene diamine N,N'- disuccinic acid, or alkali metal, or alkaline earth, ammonium or substitutes ammonium salts thereof or mixtures thereof. Ethylenediamine N,N'- disuccinic acids, especially the (S,S) isomer have been extensively described in US patent 4, 704, 233, November 3, 1987 to Hartman and Perkins. Ethylenediamine N,N'- disuccinic acid is, for instance, commercially available under the tradename ssEDDS^{®} from Palmer Research Laboratories.

Suitable amino carboxylate chelating agents useful herein include ethylene diamine tetra acetates, diethylene triamine pentaacetates, diethylene triamine pentoacetate (DTPA), N-hydroxyethylethylenediamine triacetates, nitrilotriacetates, ethylenediamine tetraproprionates, triethylenetetraaminehexaacetates, ethanoldiglycines, propylene diamine tetracetic acid (PDTA) and methyl glycine di-acetic acid (MGDA), both in their acid form, or in their alkali metal, ammonium, and substituted ammonium salt forms. Particularly suitable to be used herein are diethylene triamine penta acetic acid (DTPA), propylene diamine tetracetic acid (PDTA) which is, for instance, commercially available from BASF under the trade name Trilon FS^{®} and methyl glycine di-acetic acid (MGDA).

Further carboxylate chelating agents to be used herein include malonic acid, salicylic acid, glycine, aspartic acid, glutamic acid, or mixtures thereof.

Said chelating agents, especially phosphonate chelating agents like diethylene triamine penta methylene phosphonates, are particularly preferred in the compositions according to the present invention as they have been found to further contribute to the disinfecting properties of the compositions herein.

Typically, the compositions according to the present invention comprise up to 5% by weight of the total composition of a chelating agent, or mixtures thereof, preferably from 0.002% to 3% by weight and more preferably from 0.002% to 1.5%.

The compositions herein may further comprise a radical scavenger as a preferred optional ingredient. Suitable radical scavengers for use herein include the well-known substituted mono and di hydroxy benzenes and derivatives thereof, alkyl- and aryl carboxylates and mixtures thereof. Preferred radical scavengers for use herein include di-tert-butyl hydroxy toluene, hydroquinone, di-tert-butyl hydroquinone, mono-tert-butyl hydroquinone, tert-butyl-hydroxy anysole, benzoic acid, toluic acid, catechol, t-butyl catechol, 2-methoxy-phenol, 2-ethoxy-phenol, 4-allyl-catechol, 2-methoxy-4-(2-propenyl)phenol, benzylamine, 1,1,3-tris(2-methyl-4-hydroxy-5-t-butylphenyl) butane, as well as n-propyl-gallate. Highly preferred for use herein is di-tert-butyl hydroxy toluene, which is for example commercially available from SHELL under the trade name IONOL CP®.

Typically, the compositions according to the present invention comprise up to 5% by weight of the total composition of a radical scavenger, or mixtures thereof, preferably from 0.01 % to 1.5% by weight and more preferably from 0.01% to 1%.

The compositions herein may comprise as an optional ingredient a solvent or mixtures thereof. When used, solvents will, advantageously, give an enhanced cleaning to the compositions of the present invention. Suitable solvents for incorporation in the compositions according to the present invention include propylene glycol derivatives such as n-butoxypropanol or n-butoxypropoxypropanol, water-soluble CARBITOL® solvents or water-soluble CELLOSOLVE® solvents. Water-soluble CARBITOL® solvents are compounds of the 2-(2-alkoxyethoxy)ethanol class wherein the alkoxy group is derived from ethyl, propyl or butyl. A preferred water-soluble carbitol is 2-(2-butoxyethoxy)ethanol also known as butyl carbitol. Water-soluble CELLOSOLVE® solvents are compounds of the 2-alkoxyethoxyethanol class, with 2-butoxyethoxyethanol being preferred. Other suitable solvents are benzyl alcohol, methanol, ethanol, isopropyl alcohol and diols such as 2-ethyl-1,3-hexanediol and 2,2,4-trimethyl-1,3-pentanediol and mixture thereof. Preferred solvents for use herein are n-butoxypropoxypropanol, butyl carbitol® and mixtures thereof. A most preferred solvent for use herein is butyl carbitol®.

The solvents may typically be present within the compositions of the invention at a level up to 15% by weight, and preferably from 1% to 7% by weight of the composition.

The compositions according to the present invention formulated in their liquid form may further comprise as an optional ingredient, a shear thinning polymeric thickener or a mixture thereof.

Such shear thinning polymeric thickeners are suitable herein especially in the embodiement of the present invention wherein the compositions herein comprise a peroxygen bleach. Indeed, they perform a dual function when they are incorporated in the liquid compositions according to the present invention that comprise a peroxygen bleach, said function being not only to prevent or diminish inhalation by the user of peroxygen bleach mist/fog when such a composition is sprayed onto the surface to be disinfected, but also to provide increased contact time of the composition on vertical surfaces, thereby reducing the risk of composition dripping.

Suitable shear thinning polymeric thickeners to be used herein include synthetic and natural occurring polymers. Suitable shear thinning polymeric thickeners for used herein include polyurethane polymers, polyacrylamide polymers, polycarboxylate polymers such as polyacrylic acids and sodium salts thereof, xanthan gum or derivatives thereof, alginate or a derivative thereof, polysaccharide polymers such as substituted cellulose materials like ethoxylated cellulose, carboxymethylcellulose, hydroxymethylcellulose, hydroxypropyl cellulose, hydroxyethyl cellulose and mixtures thereof.

Preferred shear thinning polymeric thickeners for use in the compositions of the invention are xanthan gum or derivatives thereof sold by the Kelco Division of Merck under the tradenames KELTROL®, KELZAN AR®, KELZAN D35®, KELZAN S®, KELZAN XZ® and the like.

Xanthan gum is an extra cellular polysaccharide of xanthomonas campestras. Xanthan gum is made by fermentation based on corn sugar or other corn sweetener by-products. Xanthan gum comprises a poly beta-(1→4)-D-Glucopyranosyl backbone chain, similar to that found in cellulose. Aqueous dispersions of xanthan gum and its derivatives exhibit remarkable rheological properties. Xanthan gum exhibits high pseudoplasticity, i.e., over a wide range of concentrations, rapid shear thinning occurs that is generally understood to be instantaneously reversible. Preferred xanthan materials include crosslinked xanthan materials. Xanthan polymers can be crosslinked with a variety of known covalent reacting crosslinking agents reactive with the hydroxyl functionality of large polysaccharide molecules and can also be crosslinked using divalent, trivalent or polyvalent metal ions. Such crosslinked xanthan gels are disclosed in United States Patent No. 4,782,901, which patent is incorporated by reference herein. Suitable crosslinking agents for xanthan materials include metal cations such as Al+3, Fe+3, Sb+3, Zr+4 and other transition metals, etc. Known organic crosslinking agents can also be used. The preferred crosslinked xanthan agent of the invention is KELZAN AR®, a product of Kelco, a division of Merck Incorporated.

The polycarboxylate polymers for use herein preferably have a molecular weight of from 500.000 to 4.500.000, preferably from 1.000.000 to 4.000.000. Most preferred polymers for use herein contain from 0.5% to 4% by weight of a cross-linking agent, wherein the cross-linking agent tends to interconnect linear strands of the polymers to form the resulting cross-linked products. Suitable cross-linking agents include the polyalkenyl polyethers. Polycarboxylate polymers include the polyacrylate polymers. Others monomers besides acrylic acid can be used to form these polymers including such monomers as maleic anhydride which acts as a source of additional carboxylic groups. The molecular weight per carboxylate group of monomers containing a carboxylate group typically varies from 25 to 200, preferably from 50 to 150, more preferably from 75 to 125. Further other monomers may be present in the monomeric mixture, if desired, such as ethylene and propylene which act as diluents.

Preferred polycarboxylate polymers for use herein are the polyacrylate polymers. Commercially available polymers of the polyacrylate type include those sold under the trade names Carbopol®, Acrysol® ICS-1, Polygel®, and Sokalan®. Most preferred polyacrylate polymers are the copolymer of acrylic acid and alkyl (C₅-C₁₀) acrylate, commercially available under the tradename Carbopol® 1623, Carbopol® 695 from BF Goodrich, and copolymer of acrylic acid and maleic anhydride, commercially available under the tradename Polygel® DB from 3V Chemical company. Mixtures of any of the polycarboxylate polymers, herein before described, may also be used.

The compositions according to the present invention may comprise up to 10% by weight of the total composition of a shear thinning polymeric thickener, or mixtures thereof, preferably from 0.02% to 5% by weight, more preferably from 0.02% to 2% and most preferably from 0.02% to 1%.

The compositions herein may further comprise a variety of other optional ingredients such builders, buffers, stabilisers, bleach activators, soil suspenders, dye transfer agents, brighteners, perfumes, anti dusting agents, enzymes, dispersant, dye transfer inhibitors, pigments, perfumes and dyes.

### Packaging form of the disinfecting compositions

The compositions herein may be packaged in a variety of suitable detergent packaging known to those skilled in the art.

The disinfecting compositions herein in a liquid form may desirably be packaged in manually operated spray dispensing containers. Accordingly, the present invention also encompasses liquid compositions of the invention packaged in a spray dispenser, preferably in a trigger spray dispenser or in a pump spray dispenser.

For example, said spray-type dispensers allow to uniformly apply to a relatively large area of a surface to be disinfected the liquid compositions suitable for use according to the present invention; thereby contributing to the disinfecting properties of said compositions. Such spray-type dispensers are particularly suitable to disinfect vertical surfaces.

Suitable spray-type dispensers to be used according to the present invention include manually operated foam trigger-type dispensers sold for example by Specialty Packaging Products, Inc. or Continental Sprayers, Inc. These types of dispensers are disclosed, for instance, in US-4,701,311 to Dunnining et al. and US-4,646,973 and US-4,538,745 both to Focarracci. Particularly preferred to be used herein are spray-type dispensers such as T 8500® or T8900® commercially available from Continental Sprayers International or T 8100® commercially available from Canyon, Northern Ireland. In such a dispenser the liquid composition is divided in fine liquid droplets resulting in a spray that is directed onto the surface to be treated. Indeed, in such a spray-type dispenser the composition contained in the body of said dispenser is directed through the spray-type dispenser head via energy communicated to a pumping mechanism by the user as said user activates said pumping mechanism. More particularly, in said spray-type dispenser head the composition is forced against an obstacle, e.g. a grid or a cone or the like, thereby providing shocks to help atomise the liquid composition, i.e. to help the formation of liquid droplets.

The compositions of the present invention may also be executed in the form of wipes. By "wipes" it is meant herein disposable towels incorporating a disinfecting composition according to the present invention. Preferably said wipes are packaged in a plastic box. Accordingly, the present invention also encompasses wipes, e.g., disposable paper towels, incorporating a composition as described herein before. Preferably said wipes are impregnated/wetted with a liquid disinfecting composition as described herein. The advantage of this execution is a faster usage of a disinfecting composition by the user, this even outside the house, i.e., there is no need for example to pour the liquid compositions according to the present invention on the surfaces to be disinfected and to dry it out with a cloth. In other words, wipes allow disinfecting of surfaces in one step.

The present invention will be further illustrated by the following examples.

### Examples

The following compositions were made by mixing the listed ingredients in the listed proportions (weight % unless otherwise specified).

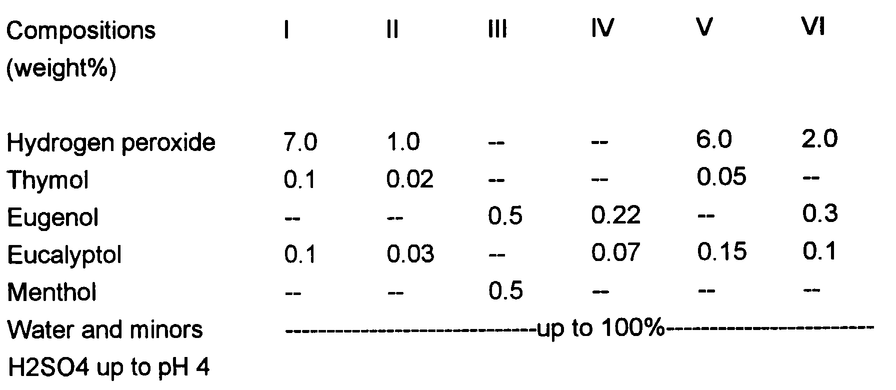

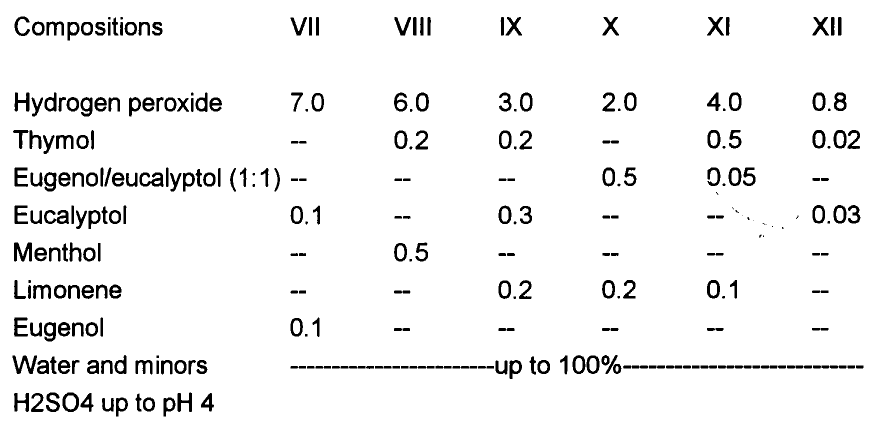

These compositions are according to the present invention. They passed the prEN 1040 test of the European committee of standardisation.

These compositions provide excellent disinfection when used neat or diluted, e.g. at 1:100, 1:25, 1:50 dilution levels, e.g., on a hard-surface, while delivering also excellent surface safety and exhibiting excellent stability upon prolonged storage periods.

## Claims

1. A disinfecting composition comprising a peroxygen bleach, from 0.003% to 5% by weight of the total composition of a cyclic terpene or a derivative thereof, and from 0.003% to 5% by weight of the total composition of a phenolic compound according to the formula: 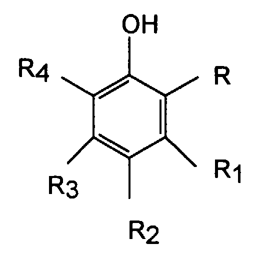 wherein R, R1, R2, R3, R4 independently are H, a linear or branched, saturated or unsaturated hydrocarbon chain having from 1 to 20 carbon atoms, an alkoxylated hydrocarbon chain according to the formula Ra(A)ₙ wherein Ra is a linear or branched, saturated or unsaturated hydrocarbon chain having from 1 to 20 carbon atoms, wherein A is butoxy, propoxy and/or ethoxy, and n is an integer of 1 to 4, or an aryl chain having from 1 to 20 carbon atoms.

2. A disinfecting composition comprising from 0.003% to 5% by weight of the total composition of a cyclic terpene or a derivative thereof and from 0.003% to 5% by weight of the total composition of eugenol, carvacrol and/or ferulic acid, said composition being free of a peroxygen bleach.

3. A process of disinfecting an inanimate surface with a composition being selected from the group consisting of:
- a composition comprising from 0.003% to 5% by weight of a cyclic terpene or a derivative thereof, and from 0.003% to 5% by weight of a phenolic compound according to the formula: 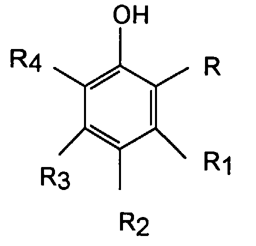 wherein R, R1, R2, R3, R4 independently are H, a linear or branched, saturated or unsaturated hydrocarbon chain having from 1 to 20 carbon atoms, an alkoxylated hydrocarbon chain according to the formula Ra(A)ₙ wherein Ra is a linear or branched, saturated or unsaturated hydrocarbon chain having from 1 to 20 carbon atoms, wherein A is butoxy, propoxy and/or ethoxy, and n is an integer of 1 to 4, or an aryl chain having from 1 to 20 carbon atoms, said composition being free of a peroxygen bleach, and
- a composition comprising a peroxygen bleach, from 0.003% to 5% by weight of a cyclic terpene or a derivative thereof and from 0.003% to 5% by weight of a phenolic compound according to the formula: 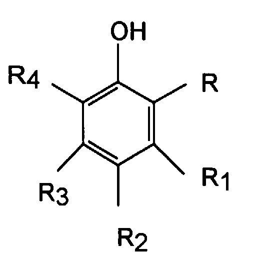 wherein R, R1, R2, R3, R4 independently are H, a linear or branched, saturated or unsaturated hydrocarbon chain having from 1 to 20 carbon atoms, an alkoxylated hydrocarbon chain according to the formula Ra(A)ₙ wherein Ra is a linear or branched, saturated or unsaturated hydrocarbon chain having from 1 to 20 carbon atoms, wherein A is butoxy, propoxy and/or ethoxy, and n is an integer of 1 to 4, or an aryl chain having from 1 to 20 carbon atoms,
said process comprising the step of applying said composition onto said surface.

4. A process of disinfecting according to claim 3 wherein said inanimate surface is a fabric, said process comprising the steps of applying said composition in its liquid form neat onto said fabric, preferably only soiled portions thereof, before rinsing, or washing then rinsing said fabric.

5. A process according to claim 4, wherein said neat composition is allowed to remain in contact with said fabric for a period of time ranging from 1 minute to 1 hour, and preferably from 1 minute to 30 minutes.

6. A process of disinfecting according to claim 3 wherein said inanimate surface is a fabric, said process comprising the steps of diluting in an aqueous bath said composition, contacting said fabrics with said aqueous bath comprising said composition, and subsequently rinsing, or washing then rinsing said fabrics.

7. A process according to claim 6, wherein the fabrics are left to soak in said aqueous bath comprising said composition for a period of time ranging from 1 minute to 48 hours, preferably from 1 minute to 24 hours.

8. A process according to claims 6 or 7, wherein said aqueous bath is formed by dissolving or dispersing a conventional laundry detergent in water.

9. A process of disinfecting according to claim 3 wherein said inanimate surface is a carpet, said process comprising the step of applying said composition onto said carpet, then optionally rubbing and/or brushing said composition into said carpet before removing said composition from said carpet.

10. A process according to claim 9 wherein said composition is removed from said carpet by mechanical means including brushing out and/or vacuum cleaning.

11. A process according to claim 3 wherein said inanimate surface is a hard-surface, said process comprising the step of applying said composition in its liquid form neat or diluted typically at a dilution level up to 100 times its weight of water, preferably into 80 to 2 times, and more preferably 60 to 10 times, before optionally rinsing said hard-surface.

12. A composition or process according to any of the preceding claims wherein said cyclic terpene or derivative thereof is selected from the group consisting of limonene, dipentene, alpha pinene, beta-pinene, menthol, terpineol, borneol, carvone, eucalyptol, camphor, cedrol, cariophyllene, verbenone, ponocarvone and mixtures thereof, and preferably is limonene, menthol, eucalyptol, terpineol or mixtures thereof.

13. A composition or process according to any of the preceding claims wherein said composition comprises from 0.005% to 3% by weight of the total composition of a cyclic terpene or a derivative thereof or a mixture thereof, and preferably from 0.05% to 1%.

14. A composition or process according to any of the preceding claims 1 or 3 to 13 wherein said phenolic compound is selected from the group consisting of eugenol, thymol, methyl salicylate, ferulic acid, carvacrol and mixtures thereof.

15. A composition or process according to any of the preceding claims 1 or 3 to 14 wherein said composition comprises from 0.005% to 3% by weight of the total composition of said phenolic compound or mixtures thereof, and preferably from 0.05% to 1%.

16. A composition or process according to any of the preceding claims 1 or 3 to 15 wherein said peroxygen bleach is hydrogen peroxide or a water soluble source thereof selected from the group consisiting of percarbonates, persilicates, persulphates, perborates, peroxyacids, dialkylperoxides, diacylperoxides, preformed percarboxylic acids, organic and inorganic peroxides, organic and inorganic hydroperoxides and mixtures thereof, and preferably is hydrogen peroxide.

17. A composition or process according to claim 16 wherein said composition comprises at least 0.01% by weight of the total composition of said peroxygen bleach, or mixtures thereof, preferably from 0.1% to 15% and more preferably from 1% to 10%.

18. A composition or process according to any of the preceding claims wherein said composition further comprises a surfactant at a level of 0.01% to 50% by weight of the total composition, preferably at a level of from 0.01% to 30%, and more preferably of from 0.1% to 20%.

19. A composition or process according to claim 18 wherein said surfactant is:
- a zwitterionic surfactant or mixtures thereof, preferably a betaine or sulphobetaine surfactant, or derivatives thereof, or mixtures thereof according to the following formula 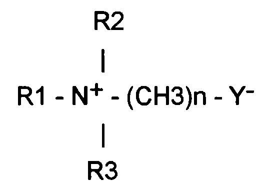 wherein R1 is a hydrocarbon chain comprising from 1 to 24 carbon atoms, preferably from 8 to 18, more preferably from 12 to 14, wherein R2 and R3 are hydrocarbon chains comprising from 1 to 3 carbon atoms, and preferably 1 carbon atom, wherein n is an integer from 1 to 10, preferably from 1 to 6 and more preferably is 1, Y is selected from the group consisting of carboxyl and sulfonyl radicals and wherein the sum of R1, R2 and R3 hydrocarbon chains is from about 14 to about 24 carbon atoms, and/or,
- an amphoteric surfactant or mixtures thereof, preferably an amine oxide having the following formula R₁R₂R₃NO wherein each of R1, R2 and R3 is independently a saturated substituted or unsubstituted, linear or branched hydrocarbon chain comprising from 1 to 30 carbon atoms, preferably R1 is a saturated substituted or unsubstituted, linear or branched hydrocarbon chain comprising from 6 to 20 carbon atoms, preferably from 8 to 16 carbon atoms, more preferably from 8 to 12, and R2 and R3 are independently substituted or unsubstituted, linear or branched hydrocarbon chains of from 1 to 4 carbon atoms, preferably of from 1 to 3 carbon atoms, and more preferably are methyl groups, or mixtures thereof.

20. A composition according to any of the claims 1, 2, or 12 to 19, which is liquid and is preferably packaged in a spray dispenser.

21. A wipe incorporating a composition according to any of the claims 1, 2 or 12 to 19.
